(19) Europäisches Patentamt
European Patent Office
Office européen des brevets

(11) Publication number: **0 040 150**
**B1**

(12) # EUROPEAN PATENT SPECIFICATION

(45) Date of publication of patent specification: **21.09.83**

(51) Int. Cl.³: **C 07 C 101/72,**
A 61 K 31/195

(21) Application number: **81400740.7**

(22) Date of filing: **08.05.81**

(54) **Fluoromethylated tyrosine methyl ester.**

(30) Priority: **09.05.80 GB 8015380**

(43) Date of publication of application:
**18.11.81 Bulletin 81/46**

(45) Publication of the grant of the patent:
**21.09.83 Bulletin 83/38**

(84) Designated Contracting States:
**BE CH DE FR IT LI NL**

(56) References cited:
**EP - A - 0 007 600**
**DE - A - 2 824 116**
**DE - A - 2 828 738**

(73) Proprietor: **MERRELL TORAUDE ET COMPAGNIE**
**16 Rue d'Ankara**
**F-67084 Strasbourg (FR)**

(72) Inventor: **Bey, Philippe**
**8 rue de Stockholm**
**F-6700 Strasbourg (FR)**
Inventor: **Jung, Michel**
**24 rue des Vergers**
**F-67400 Illkirch-Graffenstaden (FR)**

(74) Representative: **Burford, Anthony Frederick et al,**
**W.H. Beck, Greener & Co. 7 Stone Buildings**
**Lincoln's Inn**
**London WC2A 3SZ (GB)**

**EP 0 040 150 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Fluoromethylated tyrosine methyl ester

The present invention relates to a novel pharmaceutically useful methyl ester of a fluoromethylated tyrosine and provides the ester *per se* and pharmaceutical compositions comprising the ester.

We have disclosed in, for example, our Belgian Patent No. 868881 that the class of compounds of the following general Formula A are inhibitors of aromatic amino acid decarboxylase:

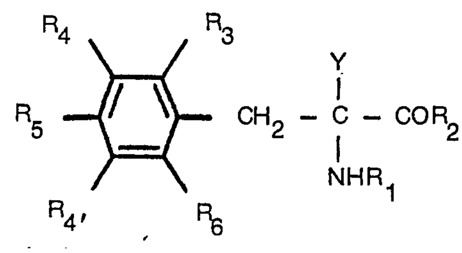

Formula A

wherein:

Y represents, *inter alia,* monofluoromethyl;

$R_1$ represents, *inter alia,* hydrogen;

$R_2$ represents, *inter alia,* hydroxy or a straight or branched chain alkoxy group of 1 to 8 carbon atoms;

$R_3$ represents, *inter alia,* hydrogen;

$R_4$ represents, *inter alia,* hydrogen;

$R_4'$ represents, *inter alia,* hydrogen;

$R_5$ represents, *inter alia,* hydroxy; and

$R_6$ represents, *inter alia,* hydrogen.

The only compound specifically disclosed in the Belgian Patent having the combination of substituents referred to above is *tert*-butyl 2-fluoromethyl-2-amino-3-(4-hydroxyphenyl)-propionate (otherwise "$\alpha$-fluoromethyl tyrosine, *tert* butyl ester"). This ester is difficult to prepare, is relatively unstable and is not amongst the particularly preferred compounds taught by the Belgian Patent.

Merck & Company have disclosed in, for example, US Patent Application No. 802391, that the class of compounds of the following general Formula B are inhibitors of amino acid decarboxylases:

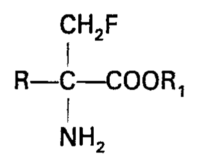

Formula B

wherein:

$R_1$ represents hydrogen or an alkyl group of 1 to 18 carbon atoms; and

R represents, *inter alia*, 4-hydroxyphenyl-methyl. The only compound specifically disclosed in the Patent Application having the combination of substituents referred to above is 2-fluoromethyl-2-amino-3-(4-hydroxyphenyl)-propionic acid (otherwise "$\alpha$-fluoromethyl tyrosine"). This acid is insoluble in water at neutral pH and is not amongst the particularly preferred compounds taught by the Patent Application.

It is well known that the catecholamine neutrotransmitters dopamine, norepinephrine and epinephrine are synthesized *in vivo* from tyrosine by a metabolic process in which an intermediate step is the decarboxylation of dopa, a reaction catalyzed by aromatic amino acid decarboxylase (AADC). Since dopamine is converted to norepinephrine, which in turn is converted to epinephrine, it is seen that the blockade of the decarboxylation step, such as by inhibition of AADC, can provide a method of regulating the levels of catecholamine neurotransmitters in body tissue.

In addition to catalyzing the decarboxylation of dopa, AADC is also known to catalyze the decarboxylation of other aromatic amino acids, namely tyrosine, phenylalanine, tryptophan and 5-hydroxytryptophan (5-HTP). Hence inhibition of AADC also provides a method for regulating *in vivo* the levels of tyramine and phenethylamine, as well as the indoleamines tryptamine and 5-hydroxytryptamine (5-HT; otherwise "serotonin").

It is believed that AADC is substrate non-specific with respect to peripheral activity, but evidence exists that a specific AADC enzyme exists in the brain for each of dopa and 5-HTP.

The prior art acknowledged *supra* teaches that, as a class, the compounds of Formulae A and B

act centrally and peripherally to affect the endogenous levels of catecholamines and indoleamines. However, 2-fluoromethyl tyrosine, methyl ester has surprisingly been found to be effective in lowering endogenous catecholamine levels in the brain but not in the heart, while endogeneous 5-HT levels in the brain and heart are substantially unaltered. This selectivity makes the methyl ester of particular use in the treatment of neuropsychiatric disorders associated with elevated brain catecholamine levels and especially as an antihypertensive agent. In particular, said selectivity reduces the occurence of gastrointestinal side effects and ortho-static hypotension usually associated with antihypertensive agents of Formulae A and B. The same degree of selectivity is not found in the corresponding acid, namely $\alpha$-fluoromethyl tyrosine, which is a weak AADC inhibitor. It is believed that the methyl ester undergoes enzymatic hydroxylation in the 3-position of the phenyl ring and ester hydrolysis to form fluoromethyl dopa *in vivo*. Fluoromethyl dopa also does not exhibit the selectivity of the methyl ester.

According to the present invention, there is provided methyl 2-fluoromethyl-2-amino-3-(4-hydroxyphenyl) propionate and pharmaceutically acceptable salts thereof. The ester has the following Formula I:

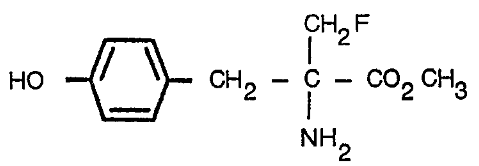

Formula I

The methyl ester of the invention has a chiral centre and therefore may occur as individual optical isomers, mixtures or racemates. It is believed that the S-isomer is the pharmacologically most active form but the invention includes the individual isomers, mixtures and racemates.

Illustrative examples of pharmaceutically acceptable salts of the ester of this invention include non-toxic acid addition salts formed with inorganic acids, such as hydrochloric, hydrobromic, sulfuric and phosphoric acids, and organic acids, such as methane sulfonic, salicylic, maleic, malonic, tartaric, citric and ascorbic acids. The salts are prepared by conventional means.

The compounds of this invention can be administered in various manners to achieve the desired effect. They can be administered alone or in the form of pharmaceutical preparations to the patient being treated either orally or parenterally, for example, subcutaneously, intravenously or intraperitoneally. They can be administered by intranasal instillation or by application to mucous membranes such as that of the nose, throat and bronchial tubes, for example, in an aerosol spray containing small particles of the compound in a spray solution or dry powder form.

The amount of the compound administered will vary and can be any effective amount. Depending on the patient, the condition being treated and the mode of administration, the quantity of compound administered can vary over a wide range to provide an effective amount in a unit dosage form. It is believed that, in human therapy, a dose in the range 0.1 to 2 mg/kg will be effective in antihypertensive treatment and a dose in the range 2.5 mg/kg to 10 mg/kg will be effective in neuropsychiatric treatment. Accordingly, it is believed that unit doses of the compound will contain 1 to 100 mg of the compound and be administered 1 to 4 times daily. It will be appreciated however that much conventional testing will have to be undertaken in order to confirm the efficacy and safety of the compound before human dose levels and regimens can be finally determined.

The term "unit dosage form" is used herein to mean a physically discrete unit containing an individual quantity of the active ingredient in admixture with or otherwise in association with the carrier, said quantity being such that one or more units are normally required for a single therapeutic administration or that, in the case of severable units such as scored tablets, at least one faction such as a half or quarter of a severable unit is required for a single therapeutic administration.

In the composition aspect of the invention there are provided pharmaceutical formulations in which form the active compounds of the invention will normally be utilized. Such formulations are prepared in a manner well known *per se* in the pharmaceutical art and usually comprise at least one active compound of the invention in admixture or otherwise in association with a pharmaceutically acceptable carrier or diluent therefor. For making these formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable diluents or carriers are well known *per se*.

The formulations of the invention may be adapted for enteral or parenteral use and may be administered to the patient in the form of tablets, capsules, suppositories, solutions, suspensions or the like.

In the specific Examples included hereinbelow illustrative examples of suitable pharmaceutical formulations are described.

The methyl ester of the invention can be prepared by esterification in manner known *per se* of $\alpha$-

fluoromethyl tyrosine. In particular, the acid can be treated with thionyl chloride to form the acid chloride which can then be subjected to alcoholysis with methanol. Alternatively, the acid can be heated in methanol saturated with hydrogen chloride gas. The acid can be prepared in manner known *per se* by hydrolysis of 2-fluoromethyl-2-amino-3-(4-methoxyphenyl)-propionitrile. In particular, the said propionitrile can be heated with hydrobromic acid under an inert atmosphere, such as for example nitrogen. The preparation of the propionitrile is described in Example 1 hereinafter.

The methyl ester produced by the foregoing processes may be isolated either *per se* or as an acid addition salt thereof.

The acid addition salts are preferably the pharmaceutically acceptable, non-toxic addition salts with suitable acids, such as those with inorganic acids, for example hydrochloric, hydrobromic, nitric, sulfuric or phosphoric acids, or with organic acids, such as organic carboxylic acids, for example, glycolic, maleic, hydroxymaleic, malic, tartaric, citric, salicylic, *o*-acetyloxybenzoic, nicotinic or isonicotinic, or organic sulfonic acids for example methane sulfonic, ethane sulfonic, 2-hydroxyethane sulfonic, toluene-p-sulfonic, or naphthalene-2-sulfonic acids.

An acid addition salt may be converted into the free methyl ester or into another acid addition salt according to methods known *per se*.

The invention is illustrated in the following Examples:

Example 1

A) 2-Amino-2-fluoromethyl-3-(4-methoxyphenyl) propionitrile

Under nitrogen, 4-methoxybenzylmagnesium chloride is prepared by adding 4-methoxybenzyl chloride (160 g) in tetrahydrofuran (THF) (800 ml) to magnesium turnings (50 g) and THF (400 ml) within about 2 hours. The reaction is initiated by the addition of a few drops of methyliodide, and the reaction flask is cooled by a bath containing water of room temperature. Stirring is continued for an additional 0.5 hour, and the solution is decanted from the excess of magnesium, transferred to a second flask and cooled to −30°C to −40°C. Fluoroacetonitrile (58 g) in THF (250 ml) is added dropwise within 40 minutes, keeping the temperature (internal) between −30°C and −40°C. After the addition, stirring is continued for 10 minutes at the same temperature and then the reaction mixture is poured into a stirred solution of sodium cyanide (100 g) and ammonium chloride (100 g) in water (2 l) and stirred for 1 hour at room temperature. Sodium chloride (400 g) is added to separate the phases. The THF layer (upper phase) is removed, and the aqueous layer is extracted with diethyl ether (3 x 1 l). After drying ($Na_2SO_4$) the THF solution and the ethereal extracts are stripped to give 200 g of crude product. Treatment of a solution of the crude nitrile in diethyl ether (3 l) with HCl gas precipitates the hydrochloride which is recrystallized from ethanol/ether (120 g).

B) 2-Amino-2-fluoromethyl-3-(4-hydroxyphenyl)propionic acid

Under nitrogen, 2-amino-2-fluoromethyl-3-(4-methoxyphenyl)-propionitrile, hydrochloride (30 g) and conc. hydrobromic acid (500 ml) are heated at 90°C for 30 hours. After evaporation, the residue is dissolved in water, and aqueous ammonia is added until pH 5.5. The precipitate is collected, dried and washed several times carefully with acetone. The washed precipitate is dissolved in 2N hydrochloric acid, treated with charcoal, and reprecipitated by addition of ammonia (pH 5.5). Reprecipitation in the same manner gives 2-amino-2-fluoromethyl-3-(4-hydroxyphenyl)propionic acid (16. 6 g), mp 239°C (dec.). Anal: calcd for $C_{10}H_{12}FNO_3$: C, 56.33, H, 5.67, N, 6.57 Found: C, 56.34, H, 5.73, N, 6.42

C) Methyl 2-amino-2-fluoromethyl-3-(4-hydroxyphenyl) propionate

With ice cooling, a suspension of 2-amino-2-fluoromethyl-3-(4-hydroxyphenyl)propionic acid (10.0 g) in absolute methanol (400 ml, dried over magnesium) is saturated with HCl gas. After heating at 90°C overnight, the solvent is removed under reduced pressure, and the residue is dried for 3 hours under the vacuum of an oil pump. After dissolving in absolute methanol (400 ml) and saturating with HCl gas, the mixture is heated at 90°C overnight again. After evaporation of the solvent, the residue is dissolved in water and a solution of sodium carbonate is added with stirring until pH 10. The suspension is extracted 3 times with ether; the combined ether extracts are filtered, washed carefully with water, dried ($Na_2SO_4$) and evaporated to give pure methyl 2-amino-2-fluoromethyl-3-(4-hydroxyphenyl)propionate (7.5 g, 70%) as white crystals, m.p. 130°C; NMR ($CD_3OD$): $\delta$ 2.77 (2H, AB, $J_{AB} = 14Hz$), 3.70 (3H, s), 4.50 (2H, ABX, $J_{AB} = 9Hz$, $J_{AX} = J_{BX} = J_{H-F} = 46Hz$), 6.87 (4H, $A_2B_2$ with additional fine splitting, $J_{AB} = 9Hz$).

Anal: Calcd for $C_{11}H_{14}FNO_3$: C, 58.14; H, 6.21; N, 6.16. Found: C, 58.32; H, 6.34; N, 6.18.

Example 2

The effects of methyl 2-amino-2-fluoromethyl-3-(4-hydroxyphenyl)propionate on AADC inhibition and catecholamine levels in various organs can be demonstrated by the following test procedure:

Male Sprague Dawley rats (100 to 180 g) are given the test compound in a suitable vehicle, either orally or by injection, each day over the period of time desired. The animals are kept on a constant 12 hour day-night cycle, and body weights are measured daily. The animals are sacrificed by decapitation 12 to 24 hours after receiving the last dose of the test compound. Control animals receive

the vehicle alone. Rats in single dose tests were fasted for 24 hours before administration of the test compound whilst rats in multiple dose tests were allowed free access to food and water.

Immediately after death, the brain and heart (where appropriate) are removed and separately homogenized either by 50 ml phosphate buffer pH 6.8 for AADC determination or in icecold 0.4 M $HClO_4$ containing 0.05% (w/v) EDTA and 0.1% (w/v) sodium bisulfite for catecholamine determination. AADC activity is determined by the $^{14}CO_2$ trapping method of J. Christenson, *Archs. Biochem. Biophys. 141,* 356 (1970) using DL-[1—$14_C$]-dopa as the substrate. Catecholamines are determined by centrifuging the homogenate, adding the supernatant to alumina buffered to pH 8.0—8.4 to absorb the catecholamines, re-extracting the catecholamines from the alumina with 2.1 M $HClO_4$ and determining the catecholamines in the extracts using reversed-phase ion-pair HPLC with electrochemical detection.

In the brain, doses of 10 mg/kg and 200 mg/kg produced a 10% reduction and a 70—80% reduction of AADC activity, respectively. AADC activity in the heart is not reduced significantly, however, up to a dose of 200 mg/kg. Brain norepinephrine was decreased 15—20% and 50% at doses of 10 mg/kg and 200 mg/kg, respectively, while dopamine was decreased significantly at a dose of 50 mg/kg and 75% at 200 mg/kg. In the heart, no reduction of norepinephrine is noted up to a dose of 200 mg/kg. When tested after multiple administration of 10, 25, or 50 mg/kg given daily for six days in rats sacrificed 24 hours after the sixth dose, the compound produced a 50% decrease in brain norephinephrine at a dose of 10 mg/kg (daily). Higher doses do not give an additional response. Brain dopamine is decreased 50% at a dose of 50 mg/kg, but not significant effect is noted at lower doses. Serotonin (5—HT) levels, as assessed by standard techniques, are not altered in the brain even at the highest dose tested. Heart norepinephrine is not significantly changed at any dose.

The inhibition is sensitive to agents affecting tyrosine hydroxylase activity. Co-administration of $\alpha$-methyl tyrosine, a well known inhibitor of tyrosine hydroxylase, reduces the extent of inhibition of AADC activity in the brain. However, in animals pretreated with haloperidol at doses known to increase dopamine turnover and brain tyrosine hydroxylase activity, the inhibition is more pronounced than in non-haloperidol treated animals.

Yohimbine, a relatively selective $\alpha_2$ antagonist, produces a decrease of norepinephrine and an increase of its metabolite MOPEG-sulfate. Combining yohimbine and the methyl ester produces a small additional decrease of norepinephrine over yohimbine up to 8 days of methyl ester treatment. However, in animals which have been treated for 2 weeks with 10 mg/kg methyl ester, the same dose of yohimbine no longer produces an additional decrease of norepinephrine. Prolonged treatment with 10 mg/kg methyl ester should result in a minimal dysfunction of the norepinephrine system under testing conditions but should stabilize it towards conditions known to increase its activity, namely emotion, stress, exercise, $\alpha_2$-antagonists etc. At higher doses of about 25 to 50 mg/kg, the dopamine system is also affected and it is predicted that at these levels there will be a neuroleptic effect or at least potentiation of known dopamine antagonists such as butyrophenones, e.g. haloperidol.

## Example 3

An illustrative composition for hard gelatin capsules is as follows:—

| | | |
|---|---|---|
| (a) | methyl ester of invention | 20 mg |
| (b) | talc | 5 mg |
| (c) | lactose | 90 mg |

The formulation is prepared by passing the dry powders of (a) and (b) through a fine mesh screen and mixing them well. The powder is then filled into hard gelatin capsules at a net fill of 115 mg per capsule.

## Example 4

An illustrative composition for tablets is as follows:—

| | | |
|---|---|---|
| (a) | methyl ester of invention | 20 mg |
| (b) | starch | 43 mg |
| (c) | lactose | 45 mg |
| (d) | magnesium stearate | 2 mg |

The granulation obtained upon mixing the lactose with the compound (a) and part of the starch and granulated with starch paste is dried, screened, and mixed with the magnesium stearate. The mixture is compressed into tablets weighing 110 mg each.

Example 5

An illustrative composition for an injectable suspension is the following 1 ml ampul for an intramuscular injection:—

| | | Weight per cent |
|---|---|---|
| (a) | methyl ester of invention | 1.0 |
| (b) | polyvinylpyrrolidone | 0.5 |
| (c) | lecithin | 0.25 |
| (d) | water for injection to make | 100.0 |

The materials (a)—(b) are mixed, homogenized, and filled into 1 ml ampuls which are sealed and autoclaved 20 minutes at 121°C. Each ampul contains 10 mg per ml of novel compound (a).

**Claims**

1. Methyl 2-fluoromethyl-2-amino-3-(4-hydroxyphenyl)propionate and pharmaceutically acceptable salts thereof.
2. A pharmaceutical composition comprising a compound as claimed in Claim 1 as an active ingredient in admixture or otherwise associated with a pharmaceutically acceptable diluent or carrier.
3. A pharmaceutical composition as claimed in Claim 2 in unit dosage form containing 1 to 100 mg of said compound per unit dose.
4. A compound as claimed in Claim 1 for use as an antihypertensive agent.
5. A compound as claimed in Claim 1 for use as a neuropsychiatric agent.

**Revendications**

1. Fluorométhyl-2 amino-2 (hydroxy-4 phényl)-3 propionate de méthyl et ses sels acceptables en pharmacie.
2. Composition pharmaceutique comprenant un composé comme revendiqué dans la revendication 1 comme ingrédient actif mélangé ou associé d'autre façon avec un diluant ou support acceptable en pharmacie.
3. Composition pharmaceutique comme revendiqué dans la revendication 2, sous forme d'une dose unitaire contenant 1 à 100 mg dudit composé par dose unitaire.
4. Composé selon la revendication 1, pour l'emploi comme agent antihypertensif.
5. Composé comme revendiqué dans la revendication 1, pour l'emploi comme agent neuropsychiatrique.

**Patentansprüche**

1. Methyl-2-fluormethyl-2-amino-3-(4-hydroxyphenyl)-propionat und seine pharmazeutisch verträglichen Salze.
2. Pharmazeutische Zubereitung, enthaltend eine Verbindung nach Anspruch 1 als wirksamen Bestandteil im Gemisch oder in einer sonstigen Vermengung mit einem pharmazeutisch verträglichen Verdünnungsmittel oder Trägerstoff.
3. Pharmazeutische Zubereitung nach Anspruch 2 in Form einer Dosierungseinheit, enthaltend 1 bis 100 mg der genannten Verbindung pro Dosierungseinheit.
4. Verbindung nach Anspruch 1 zur Anwendung als Mittel gegen Bluthochdruck.
5. Verbindung nach Anspruch 1 zur Anwendung als neuropsychiatrisches Mittel.